(19) Europäisches Patentamt
European Patent Office
Office européen des brevets

(11) Veröffentlichungsnummer: **0 191 344**
**B1**

(12) # EUROPÄISCHE PATENTSCHRIFT

(45) Veröffentlichungstag der Patentschrift: **07.01.88**

(51) Int. Cl.⁴: **C 07 C 43/29**, C 07 C 49/84, C 07 C 147/00, C 08 G 65/40

(21) Anmeldenummer: **86101029.6**

(22) Anmeldetag: **25.01.86**

(54) **Bromhaltige oligomere Ether von Bis-chlormethyl-diphenylethern.**

(30) Priorität: **07.02.85 DE 3504169**

(43) Veröffentlichungstag der Anmeldung: **20.08.86 Patentblatt 86/34**

(45) Bekanntmachung des Hinweises auf die Patenterteilung: **07.01.88 Patentblatt 88/1**

(84) Benannte Vertragsstaaten: **CH DE FR GB IT LI**

(56) Entgegenhaltungen:
**EP - A - 0 018 632**
**DE - A - 2 021 830**
**DE - A - 2 636 027**
**FR - A - 2 369 312**

(73) Patentinhaber: **BAYER AG, Konzernverwaltung RP Patentabteilung, D-5090 Leverkusen 1 Bayerwerk (DE)**

(72) Erfinder: **Fuhr, Karl, Dr., Krüllsdyk 55, D-4150 Krefeld (DE)**
Erfinder: **Müller, Friedemann, Dr., Am Steinacker 5, D-4040 Neuss 21 (DE)**
Erfinder: **Eicher, Theo, Dr., Waldstrasse 7, D-7000 Stuttgart 70 (DE)**

EP 0 191 344 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft bromhaltige Oligomere mit Benzylaryl- und Diaryletherstrukturen und ihre Verwendung als flammhemmende Mittel in Kunststoffen.

Die bromhaltigen Oligomeren werden erhalten, wenn man bromhaltige Bisphenole mit Bis-chlormethyl-diphenyl-ethern in inerten Lösungsmitteln in Gegenwart von Katalysatoren unsetzt. Zur Begrenzung des Molekulargewichts werden monofunktionelle Phenole und/oder monofunktionelle Chlormethylverbindungen in kleinen Mengen mit umgesetzt.

Als Flammschutzmittel werden im allgemeinen 5-25 Gewichtsprozent, bezogen auf das Gesamtgemisch, an bromhaltigen Oligomeren eingesetzt. Bevorzugt werden sie mit synergistisch wirkenden Metalloxiden verwendet, wie Antimon(III)-oxid, Zinn(IV)-oxid, Wismut(III)-oxid. Dabei wird die Brandklasse V-2 bis V-0 nach UL 94 erhalten. Besonders bemerkenswert ist dabei, dass im Vergleich zu herkömmlichen monomeren Bromverbindungen ca. ein Drittel der üblicherweise benötigten Brommenge bei gleicher Wirkung eingespart wird.

Man hat bereits Kunststoffen zur Verminderung ihrer Brennbarkeit halogenhaltige Mittel, insbesondere organische Brom- und/oder Chlorverbindungen, zusammen mit synergistisch wirkenden Metalloxiden zugesetzt. Für eine ausreichende Wirksamkeit, z.B. die Brandklasse V-2 bis V-0 (Prüfkörper, 2,5 mm dick) nach UL 94, sind bei Kunststoffen auf Styrolbasis in Gegenwart von 3-5 Gewichtsprozent Antimon(III)-oxid ca. 15 Gewichtsprozent einer hochbromierten aromatischen Verbindung wie Decabrom- oder Octabromdiphenylether (Bromgehalte um 80 Gewichtsprozent) erforderlich (siehe Taschenbuch der Kunststoff-Additive, Carl Hanser Verlag München Wien 1979, Seiten 408 und 409).

Diese grossen Mengen niedermolekularer Zusätze verschlechtern aber durch Weichmachereffekte, Auswandern und Verdampfen, bei Verarbeitung bzw. Lagerung und Gebrauch, die Eigenschaften der Kunststoffe.

Aus EP-A 18 631 und EP-A 18 632 ist bekannt, bestimmte oligomere bromhaltige Diarylether für flammwidrige Kunststoffe zu verwenden, die in Dimethylacetamid und Chlorbenzol aus Bisphenolaten und aromatischen Polybromverbindungen hergestellt worden sind. Diese Herstellungsweise ist aufwendig und führt durch Bildung in Alkali- oder Erdalkalibromid zu einer Verringerung der wirksamen Brommenge.

In der US-PS 3075949 und den DE-OS 2650117, 2929914 und 3214199 werden bromhaltige Umsetzungsprodukte aus Xylylendichloriden (auch halogenhaltig) mit halogenierten Bisphenolen als Flammschutzadditive beschrieben. Mit diesen wird in üblichen Kunststoffen schnell die Grenze der Verträglichkeit überschritten, so dass es zum Verlust der mechanischen Festigkeit kommt. Ausserdem sind Xylylendichloride schwer zugänglich.

Die erfindungsgemässen Oligomeren vermeiden diese Nachteile. Im Gegensatz zu den Xylylendichloriden sind Bischlormethyl-diphenylether leicht und mit praktisch 100%iger Ausbeute zugänglich. Ausserdem weisen die erfindungsgemässen Oligomeren durch die Diphenyletherstruktureinheiten gegenüber Kunststoffen eine allgemein grössere Verträglichkeit auf. Aufgrund ihres günstigen Schmelzbereiches sind sie leichter einzuarbeiten.

Gegenstand der Erfindung sind somit bromhaltige Oligomere, die zur Begrenzung ihres Molekulargewichtes Phenyl-, Biphenyl- bzw. Naphtyl- und/oder Benzyl-, Phenoxibenzyl- bzw. Menaphthyl-endgruppen tragen können, folgender Struktur:

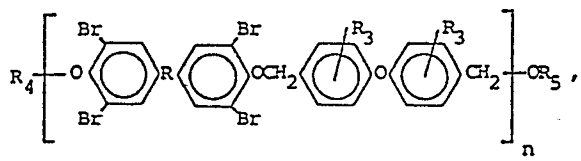

wobei

R eine 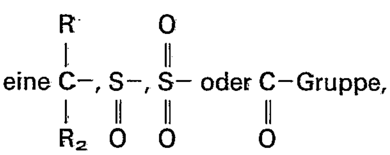 -Gruppe,


ein Sauerstoff- oder Schwefelatom, bzw. eine direkte Bindung,

$R_1$ und $R_2$ Wasserstoffatome, jeweils eine Alkylgruppe mit 1 bis 4 Kohlenstoffatomen (diese können auch miteinander verbunden sein),

$R_3$ Wasserstoffatome oder Alkylgruppen mit 1 bis 4 Kohlenstoffatomen,

$R_4$ und $R_5$ jeweils

1. eine 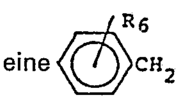 - und eine 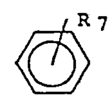 -Gruppe,

oder 2. eine 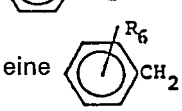 - und eine

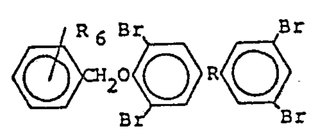 -Gruppe,

oder 3. eine 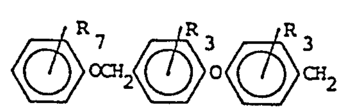

- und eine 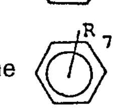 -Gruppe,

$R_6$ Wasserstoffatome, Alkylgruppen mit 1 bis 4 Kohlenstoffatomen oder eine Phenoxigruppe und

$R_7$ Wasserstoffatome, Alkylgruppen mit 1 bis 4 Kohlenstoffatomen eine Phenylgruppe und/oder Halogenatome und

n eine ganze Zahl in 2-15; bevorzugt in 5-12, bedeuten.

Bei den Substituenten $R_4$ und $R_5$ kann in den Endgruppen die Phenyl- durch eine Naphthylgruppe ersetzt sein.

Unter Menaphthyl versteht man einen Rest, der durch Entfernung eines Wasserstoffs aus der Methylgruppe eines Methylnaphthalins entsteht.

Die erfindungsgemässen bromhaltigen Oligomeren mit Benzylaryl- und Diarylethereinheiten können erhalten werden, indem man in einem Lösungsmittel, wie Methanol, Isopropanol, Dioxan, Toluol, Methylenchlorid, Perchlorethylen oder in Lösungsmittelgemischen, ein Bisphenolat mit der Bis-chlormethyl-Verbindung in Gegenwart eines geeigneten Katalysators bei Temperaturen um 80° C umsetzt. Um funktionelle Endgruppen zu vermeiden und die gewünschte Molekülgrösse einzustellen, müssen zusätzlich ein monofunktionelles Phenol als Phenolat und/oder eine Monochlormethyl-Verbindung an der Reaktion teilnehmen.

Wird ein Alkohol als Lösungsmittel verwendet, so fällt das gewünschte Produkt als feinkörniges farbloses Granulat an (Fällungspolykondensation). Anorganische Bestandteile, z.B. Natriumchlorid, können durch Wasser entfernt werden. Anschliessend wird im Vakuum bei 120° C getrocknet.

Die erfindungsgemässen Verbindungen können auch in Lösungsmitteln für das Oligomer, z.B. Dioxan oder Toluol, und besonders zweckmässig nach dem Verfahren der Grenzflächenkondensation hergestellt werden. Als Lösungsmittel werden zweckmässig ein chlorierter Kohlenstoff, wie z.B. Perchlorethylen, und Wasser verwendet. Auch in diesem Fall empfiehlt sich die Mitverwendung eines Katalysators. Zur Aufarbeitung wird das Reaktionsgemisch wie bei der Herstellung in Lösung in Methanol oder Aceton gegeben, das ausgefallene Produkt abefiltriert, ausgewaschen und getrocknet.

Beispiele für Bisphenole sind 2,2-(4-Hydroxy-3,5-dibrom)propan (Tetrabrombisphenol A), 4,4'-Dihydroxi-3,3',5,5'-tetrabromdiphenylsulfoxid bzw. -sulfon, sowie 4,4'-Dihydroxi-3,3',5,5'-tetrabrombenzophenon und 4,4'-Dihydroxi-3,3'-5,5'-tetrabrom-diphenylthioether. Als Beispiele für die erfindungsgemässen Bis-chlormethyl-Verbindungen werden 2,4'- oder 4,4'-Bis-chlormethyl-diphenylether und ihre technischen Gemische sowie Bis-chlormethyl-ditolylether angegeben. Beispiel für Phenole sind Phenol, Kresole, Xylenole, Naphthole, Monohydroxidiphenyle und deren Halogenderivate, wie z.B. Tribromphenol. Beispiele für Monochlormethyl-Verbindungen sind Benzylchlorid, Menaphthylchlorid und Chlormethyldiphenylether sowie deren Alkylderivate wie z.B. 4-Methylbenzylchlorid.

Als Katalysatoren zur Herstellung oligomerer und polymerer Ether mit Benzylphenyletherstrukturen sind Trialkylamine, Tetraalkylammoniumhalogenide und Tetraalkylphosphoniumhalogenide geeignet. Besonders wirkungsvoll ist Tetrabutylphosphoniumbromid.

Ebenfalls Gegenstand der Erfindung ist die Verwendung der beschriebenen bromhaltigen Oligomeren als flammhemmende Mittel in Kunststoffen, wobei sie gegebenenfalls zusammen mit synergistisch wirkenden Metalloxiden, wie Antimon(III)-oxid, organischen Phosphorverbindungen, wie Triphenylphosphat bzw. Methanphosphonsäurediphenylester, und weiteren flammhemmenden bromhaltigen Verbindungen, wie Octa- und Decabromdiphenylether, in einer Menge von 5-25 Gewichtsprozent, bezogen auf die gesamte Mischung, eingesetzt werden.

Ein typischer ABS-Kunststoff zeigt so überraschend bei Zusatz von 20,0 Gewichtsprozent der Oligomeren aus Tetrabrombisphenol A, 4,4'-Bischlormethyl-diphenylether, Phenol, und Benzylchlorid in Gegenwart von 3,0% Antimon(III)-oxid bei einem Bromgehalt von 8,1 Gewichtsprozent im UL-Test ein Brandverhalten von V-0. Bei Verwendung von Octabromdiphenylether werden für V-0 12,0% Brom benötigt.

Als Kunststoffe sind besonders geeignet Thermoplasten wie Polystyrol, Co- und Pfropfpolymerisate mit Styrol, z.B. Styrol/Acrylnitril-Co-polymerisate, hochschlagfestes Polystyrol, Pfropfpolymerisate von Styrol, Acrylnitril und Kautschuken (vom ABS-Typ), Polyethylen, Polypropylen, Polyvinylchlorid, Polyacrylat, Polyester, aromatisches Polycarbonat, Polyamid, Polyurethan, Polysulfon, Polyphenylenoxid, Polyphenylensulfid oder auch Mischungen daraus, sowie duroplastische Kunststoffe, z.B. aus ungesättigten Polyesterharzen, Epoxidharzen und vernetzbaren Polyurethanrohstoffen.

Diese Kunststoffe können neben den erfindungsgemässen und weiteren Flammschutzmitteln, falls erforderlich, Zusätze wie Füllstoffe, Pigmente und Glasfasern enthalten. Dieses gilt auch für typische und notwendige Hilfsstoffe, z.B. Hitzestabilisatoren, Lichtschutzmittel, Antioxidantien, Gleit- und Schmierstoffe, Entformungsmittel und Farbstoffe.

Beispiele für weitere halogenhaltige Flammschutzmittel sind Hexabrombenzol, Pentabromtoluol, Octa- und Decabromdiphenyl, Hexabrom bis Decabromdiphenylether, Hexabrom-bis-phenoxiethan und Ethylen-bis-tetrabromphthalimid sowie Gemische aus ihnen.

Als weitere hochwirksame Flammschutzmittel kommen als synergistisch wirkende Metalloxide z.B. Antimon(III)-oxid, Blei(IV)-oxid, Cer(IV)-oxid, Kupfer(II)-oxid, Molybdän(VI)-oxid, Vanadium(V)-oxid, Wismut(III)-oxid, Wolfram(VI)-oxid, Zinn(IV)-oxid und Zirkon(IV)-oxid in Betracht, sowie deren Mischungen.

Phosphorhaltige Flammschutzmittel sind z.B. Triphenylphosphat, Diphenylkresylphosphat, Methandiphenylphosphonat und Triphenylphosphit. Die Mengen liegen in den üblichen Bereichen.

Beispiel für typische Füllstoffe und Pigmente sind Glaskugeln, Quarzmehl, Kalk, Kreide, Leicht- und Schwerspat, Aluminiumhydroxid, Aluminiumoxid, Magnesium- und Aluminiumsilikate wie Glimmer, Talkum oder Kaolin, Aluminiumphosphat, Calciumphosphat, Zinkborat, Zinkoxid,

Eisenoxid, Titandioxid, Kohlenstoff, z.B. als Graphit oder Russ, sowie faserförmige Stoffe, z.B. Glasfasern.

*Beispiel 1:*

Zu 900 ml Isopropanol werden 136,0 g Tetrabrombisphenol A (0,25 Mol), 20,0 g Ätznatron (0,50 Mol) und 4,0 g Tetrabutylphosphoniumbromid gegeben. Das auf 80° C erwärmte Gemisch versetzt man daraufhin unter gutem Rühren in 6 Stunden mit einer ca. 60° C warmen Lösung aus 250 ml Isopropanol, 54,4 g Bis-chlormethyl-diphenylether (0,40 Äquivalent Chlor bei einem Chlorgehalt von 26,0 Gewichtsprozent) und 12,7 g Benzylchlorid (0,10 Mol).

Schon nach kurzer Zutropfdauer bildet sich das gewünschte Oligomer als feinkörniges, reinweisses Granulat. Die Nachreaktionsdauer beträgt 12 Stunden.

Nach Abkühlen wird der Feststoff abfiltriert und mehrmals mit 60° C warmem Wasser chloridfrei gewaschen. Anschliessen wird das Produkt über Nacht im Vakuumtrockenschrank bei 60-70° C getrocknet.

Die Ausbeute beträgt 167,0 g oder 90,3 Prozent der Theorie. Das Produkt schmilzt bei 220-230° C. Der Bromgehalt beträgt 40,6 Gewichtsprozent (theoretisch 43,3 Gewichtsprozent).

Zur Prüfung auf die Flammschutzwirkung des zuvor beschriebenen bromhaltigen Präparats werden 195,0 g zusammen mit 39,0 Antimon(III)-oxid in 1066,0 g eines typischen ABS-Copolymerisats (Novodur PX der Bayer AG) auf einem Kneter bei 210-230° C eingearbeitet. Aus dem erhaltenen Material werden dann für den UL-Test geeignete Prüfkörper von 2,5 mm Dicke hergestellt. Es enthält neben den 3,0 Gewichtsprozent Antimon(III)-oxid 6,1 Gewichtsprozent an den erfindungsgemässen oligomeren Polyether gebundenes Brom. Der UL-Test ergibt das Prüfergebnis V-2.

Werden dagegen 260,0 g obigen Präparats, 39,0 g Antimon(III)-oxid und 1001,0 g ABS-Copolymerisat (Novodur PX) eingesetzt, so enthält das Gemisch 8,1 Gewichtsprozent Brom. Der UL-Test ergibt das Prüfergebnis V-0 (2,5 mm dick).

*Vergleichsversuch*

Werden 195,0 g Octabromdiphenylether zusammen mit 39,0 g Antimon(III)-oxid in 1066,0 g ABS-Copolymerisat (Novodur PX) eingesetzt, zu für den UL-Test geeigneten Prüfkörpern von 2,5 mm Dicke verarbeitet, so ist das Prüfergebnis im UL-Test V-2. Der Bromgehalt beträgt dabei 12,0%.

*Beispiel 2:*

Zu 1000 ml Isopropanol werden 145,0 g Tetrabrombisphenol A (0,27 Mol), 22,1 g Tribromphenol (0,07 Mol), 24,0 g Ätznatron (0,60 Mol) und 4 g Tetrabutylphosphoniumbromid gegeben. Das auf 80° C erwärmte Gemisch versetzt man daraufhin in 6 Stunden unter gutem Rühren mit einer 60° C warmen Lösung aus 250 ml Isopropanol, 81,8 g Bis-chlormethyl-diphenylether (0,60 Äquivalent Chlor bei einem Chlorgehalt von 26,0 Gewichtsprozent). Die Nachreaktionsdauer beträgt 12 Stunden. Die Aufarbeitung erfolgt wie im Beispiel 1. Die Trocknung wird über Nacht im Vakuumtrockenschrank bei 60-70° C durchgeführt.

Die Ausbeute beträgt 210,0 g eines weissen körnigen Produktes oder 93,2 Prozent der Theorie. Das Produkt schmiltz bei 200-220° C. Der Bromgehalt beträgt 42,4 Gewichtsprozent (theoretische 44,9 Gewichtsprozent).

Bei der Prüfung auf Flammschutzwirkung werden Ergebnisse entsprechend Beispiel 1 erhalten.

*Beispiel 3:*

Zu 1000 ml Isopropanol werden 145,0 g Tetrabrombisphenol A (0,27 Mol), 11,0 g Tribromphenol (0,03 Mol), 22,6 g Ätznatron (0,5 Mol) und 4 g Tetrabutylphosphoniumbromid gegeben. Das auf 80° C erwärmte Gemisch versetzt man daraufhin in 6 Stunden mit einer 60° C warmen Lösung aus 250 ml Isopropanol, 72,7 g Bis-chlormethyl-diphenylether (0,53 Äquivalent Chlor bei einem Chlorgehalt von 26,0 Gewichtsprozent) und 4,2 g Benzylchlorid (0,03 Mol).

Die Nachreaktionsdauer beträgt 12 Stunden. Die Aufarbeitung erfolgt wie im Beispiel 1. Die Trocknung wird über Nacht im Vakuumtrockenschrank bei 60-70° C durchgeführt.

Die Ausbeute beträgt 178,0 g eines weissen körnigen Produktes oder 84,4 Prozent der Theorie. Das Produkt schmilzt bei 220-230° C. Der Bromgehalt beträgt 41,6 Gewichtsprozent (theoretisch 44,2 Gewichtsprozent.

Bei der Prüfung auf Flammschutzwirkung werden Ergebnisse entsprechen Beispiel 1 erhalten.

*Beispiel 4:*

Zu 1000 ml Wasser werden 136,0 g Tetrabrombisphenol A (0,25 Mol), 20,0 g Ätznatron (0,50 Mol), 4,0 g Tetrabutylphosphoniumbromid und 20,0 g Natriumlaurylsulfat gegeben. Die auf 80° C erwärmte Lösung versetzt man daraufhin unter intensivem Rühren in 2 Stunden mit einer ca. 60° C warmen Lösung aus 54,4 g Bis-chlormethyl-diphenylether (0,40 Äquivalent Chlor bei einem Chlorgehalt von 26,0 Gewichtsprozent, 12,7 g Benzylchlorid (0,10 Mol) und 500 ml Perchlorethylen. Die Nachreaktionsdauer beträgt 6 Stunden.

Nach Abkühlen wird das Reaktionsgemisch unter Rühren in 5 l Methanol gegeben. Das ausgefallene Oligomere wird abfiltriert, zur Entfernung von Kochsalz und Emulgator mit Wasser gut gewaschen und über Nacht im Vakuumtrockenschrank bei 60-70° C getrocknet.

Die Ausbeute beträgt 171,1 g oder 92,5 Prozent der Theorie. Das Produkt schmilzt bei 220-235° C.

Bei der Prüfung auf Flammschutzwirkung werden Ergebnisse entsprechen Beispiel 1 erhalten.

## Patentansprüche

1. Bromhaltige oligomere Ether mit Benzylaryl- und Diarylstrukturen, die zur Begrenzung ihres Molekulargewichtes Phenyl-, Biphenyl- bzw. Naphthyl- und/oder Benzyl-, Phenoxibenzyl- bzw. Menaphthylendgruppen tragen, folgender Struktur:

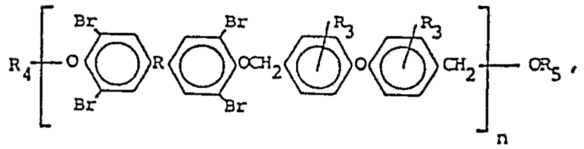

wobei

R eine 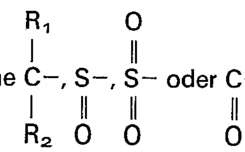 oder -Gruppe,

ein Sauerstoff- oder Schwefelatom, bzw. eine direkte Bindung,

$R_1$ und $R_2$ Wasserstoffatome, jeweils eine Alkylgruppe mit 1 bis 4 Kohlenstoffatomen (diese können auch miteinander verbunden sein),

$R_3$ Wasserstoffatome oder Alkylgruppen mit 1 bis 4 Kohlenstoffatomen,

$R_4$ und $R_5$ jeweils

1. eine 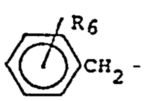 - und eine 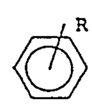 -Gruppe,

oder 2. eine 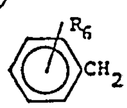 - und eine

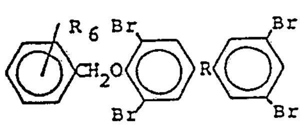 -Gruppe,

oder 3. eine 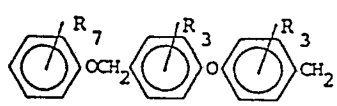

- und eine 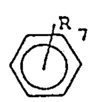 -Gruppe,

$R_6$ Wasserstoffatome, Alkylgruppen mit 1 bis 4 Kohlenstoffatomen oder eine Phenoxigruppe und

$R_7$ Wasserstoffatome, Alkylgruppen mit 1 bis 4 Kohlenstoffatomen eine Phenylgruppe und/oder Halogenatome und

n eine ganze Zahl von 2-15

bedeuten. Bei den Substituenten $R_4$ und $R_5$ kann in den Endgruppen die Phenyl- durch eine Naphthylgruppe ersetzt sein.

2. Verwendung von bromhaltigen oligomeren Ethern nach Anspruch 1 als Flammschutzmittel für Kunststoffe in Mengen von 5-25 Gewichtsprozent.

## Claims

1. Bromine-containing oligomeric ethers with benzylaryl and diaryl structures which carry phenyl, biphenyl or naphthyl and/or benzyl, phenoxybenzyl or menaphthyl end groups in order to limit their molecular weight and have the following structure:

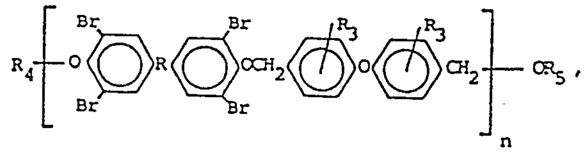

wherein

R denotes a 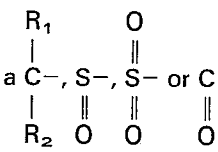 or group,

an oxygen or sulphur atom, or a direct bond, $R_1$ and $R_2$ denote hydrogen atoms or in each case an alkyl group with 1 to 4 carbon atoms (these can also be bonded to each other),

$R_3$ denotes hydrogen atoms or alkyl groups with 1 to 4 carbon atoms,

$R_4$ and $R_5$ in each case denote

1. a 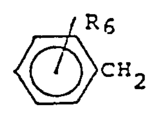 and a 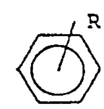 group,

or

2. a 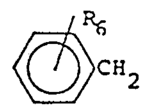

and a 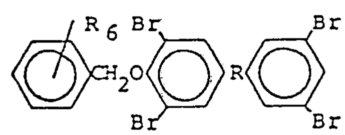 group,

or

3. a 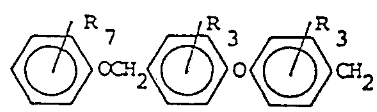

and a 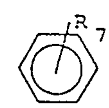 group,

$R_6$ denotes hydrogen atoms, alkyl groups with 1 to 4 carbon atoms or a phenoxy group and

$R_7$ denotes hydrogen atoms, alkyl groups with 1 to 4 carbon atoms a phenyl group and/or halogen atoms and

n denotes an integer from 2-15.

In the case of substituents $R_4$ and $R_5$ the phenyl group can be replaced by a naphthyl group in the end groups.

2. Use of bromine-containing oligomeric ethers according to Claim 1 as flame-proofing agents for plastics in quantities of 5-25 per cent by weight.

## Revendications

1. Ethers oligomères bromés à structure benzylaryliques et diaryliques portant, pour limiter leur poids moléculaire, des groupes terminaux phényle, biphényle et respectivement naphtyle et/ou benzyle, phénoxybenzyle ou ménaphtyle, à la structure suivante

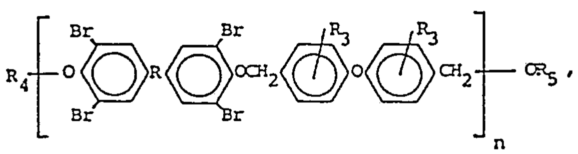

dans laquelle

R représente un groupe
$$\begin{array}{c} R_1 \\ | \\ C- \\ | \\ R_2 \end{array},\ \begin{array}{c} S- \\ \| \\ O \end{array},\ \begin{array}{c} O \\ \| \\ S- \\ \| \\ O \end{array}\ \text{ou}\ \begin{array}{c} C, \\ \| \\ O \end{array}$$
un atome d'oxygène ou de soufre, ou une liaison directe,
$R_1$ et $R_2$ représentent des atomes d'hydrogène ou chacun un groupe alkyle en $C_1$-$C_4$ (qui peuvent également être reliés entre eux),
$R_3$ représente des atomes d'hydrogène ou des groupes alkyle en $C_1$-$C_4$,
$R_4$ et $R_5$ représentent chacun

1. un groupe 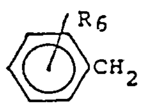 et un groupe 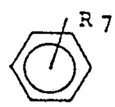

ou bien

2. un groupe 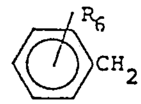

et un groupe 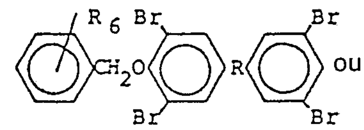 ou bien

3. un groupe 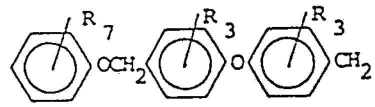

et un groupe 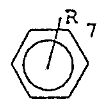

$R_6$ représente des atomes d'hydrogène, des groupes alkyle en $C_1$-$C_4$ ou un groupe phénoxy et
$R_7$ représente des atomes d'hydrogène, des groupes alkyle en $C_1$-$C_4$, un groupe phényle et/ou des atomes d'halogènes et
n est un nombre entier de 2 à 15.

Dans le cas des substituants $R_4$ et $R_5$, et dans les groupes terminaux, le groupe phényle peut être remplacé par un groupe naphtyle.

2. Utilisation des éthers oligomères bromés selon la revendication 1 en tant qu'agents ignifugeants pour des résines synthétiques, en quantités de 5 à 25% en poids.